# EUROPÄISCHE PATENTSCHRIFT

(11) **EP 2 526 085 B1**
(45) Veröffentlichungstag und Bekanntmachung des Hinweises auf die Patenterteilung: **12.08.2015**
(21) Anmeldenummer: 11700347.5
(22) Anmeldetag: 18.01.2011
(51) Int. Cl.: C07C 263/04, B01D 3/14

(54) **VERFAHREN ZUR HERSTELLUNG VON ISOCYANATEN DURCH THERMISCHE SPALTUNG VON CARBAMATEN**
METHOD FOR PRODUCING ISOCYANATES BY THERMALLY SPLITTING CARBAMATES
PROCÉDÉ DE PRODUCTION D'ISOCYANATES PAR CRAQUAGE THERMIQUE DE CARBAMATES

(30) Priorität: 19.01.2010 EP 10151089
(43) Veröffentlichungstag der Anmeldung: 28.11.2012
(73) Patentinhaber: BASF SE, 67056 Ludwigshafen (DE)
(72) Erfinder: BOCK, Michael, 67152 Ruppertsberg (DE); FRANZKE, Axel, 68161 Mannheim (DE); BAUMANN, Robert, 68529 Mannheim (DE); STROEFER, Eckhard, 68163 Mannheim (DE)
(86) Internationale Anmeldenummer: PCT/EP2011/050557
(87) Internationale Veröffentlichungsnummer: WO 2011/089098

(56) Entgegenhaltungen:
- EP-A1- 0 092 738
- EP-A1- 0 795 544
- JP-A- 2 250 857

## Beschreibung

Die Erfindung betrifft ein Verfahren zur Herstellung von Isocyanaten durch thermische Spaltung von Carbamaten, auch als Carbamidsäureester oder Urethane bekannt.

Die Carbamatspaltung gewinnt zunehmend an Bedeutung als phosgenfreies Verfahren zur Herstellung von Isocyanaten. Zur technischen Durchführung der Carbamatspaltung wurden unterschiedliche Apparate vorgeschlagen, insbesondere Kolonnen (in EP 0 795 543), Wirbelschichtreaktoren (in EP 555 628 und in DE 199 07 648), Fallfilm- oder Dünnschichtverdampfer (in EP 0 092 738). Die Carbamatspaltung kann in der Flüssig- oder in der Gasphase betrieben werden.

Problematisch bei der thermischen Spaltung von Carbamaten ist die Bildung von hochmolekularen Nebenkomponenten, die durch Weiterreaktion der Spaltprodukte mit sich selbst oder den Ausgangsstoffen entstehen. Diese können zu Anlagerungen in den Apparaten führen, damit den kontinuierlichen Betrieb einschränken und zu Ausbeuteverlusten führen. Die Rückstände enthalten insbesondere Allophanate und Isocyanurate. Die Nebenprodukte entstehen auch durch die Reaktion von Halburethanen (Semicarbamaten, d.h. eine Urethan- und eine Isocyanatfunktion enthaltende difunktionale Verbindung, Intermediate der Spaltung von Bisurethanen) mit sich selbst.

Um diese Probleme zu vermeiden, müssen die Spaltprodukte Isocyanat und Alkohol aus dem Carbamat-Spaltgas möglichst schnell voneinander getrennt werden.

Es ist weiterhin bekannt, dass die Problematik der Rück- und Weiterreaktionen im Laufe der Spaltung reduziert wird, indem die Carbamat-Spaltung in Gegenwart von Lösungsmitteln durchgeführt wird, da die Reaktionsgeschwindigkeit der Rückreaktion von Isocyanat und Alkohol (Urethanisierung) sowie auch der Weiterreaktionen bekanntermaßen von der Art des Lösungsmittels und der Verdünnung durch das Lösungsmittel abhängig ist. Beispielsweise finden sich in J.H. Saunders und K.C. Frisch: Polyurethanes, Chemistry and Technology, 1962, S. 146, Tabelle 10, Angaben über die Reaktivität von Isocyanaten mit Alkoholen in Gegenwart unterschiedlicher Lösungsmittel.

Durch Verdünnen der Carbamatspaltprodukte mit einem inerten Lösungsmittel wird die Bildung hochmolekularer Folgeprodukte zurückgedrängt; gleichzeitig dient das Lösungsmittel zur Ausschleusung dieser Nebenkomponenten und das Apparate-Fouling wird reduziert.

Die JP-2 250857 beschreibt ein Verfahren zur Herstellung von Isocyanaten durch thermische Spaltung von Carbamaten und destillative Auftrennung des Reaktionsgemisches der Carbamatspaltung in einer Kolonne mit einem Verstärkungsteil und einem Abtriebsteil, in Gegenwart eines inerten Lösungsmittels, bei welchem der Alkohol als Kopfprodukt und das Isocyanat als Sumpfprodukt abgezogen wird. Das Lösungsmittel kann bevorzugt Cyclohexylbenzol sein, das dampfförmig in den unteren Bereich der Kolonne zugeführt wird.

Die EP-B 0 795 543 beschreibt besonders geeignete Lösungsmittel zur thermischen Spaltung von Carbamaten, die einen definierten Siedepunkt oder aber einen engen Siedebereich aufweisen, und die als Destillationsschnitt von thermostabilen Flüssigkeiten gewonnen werden, ausgewählt aus der Gruppe der ortho-, meta- und para-Isomeren von Phenoxybiphenyl. Durch Einsatz derartiger Lösungsmittel beim thermischen Spalten von Carbamaten in Kolonnen kann die Sumpftemperatur der Kolonne, bei gleicher Spaltleistung und unveränderter mittlerer Temperatur im Reaktionsteil verringert werden, wodurch die Bildung von Neben- und Crackprodukten im Kolonnensumpf deutlich reduziert wird. Nachteilig an diesem Verfahren ist, dass der Rücklauf am Kolonnenkopf größtenteils aus Alkohol besteht und dass Phenoxybiphenyl kommerziell kaum verfügbar und damit teuer ist.

Es ist auch bekannt, die Geschwindigkeit der Urethanbildung durch Zusatz von Inhibitoren zu reduzieren. Als Inhibitoren der Urethanbildung sind beispielsweise Salzsäure, Benzoylchlorid oder p-Toluolsulfonsäure bekannt (vgl. Örtel: Polyurethane, 2. Auflage, 3.4.2, S. 92)

Es war demgegenüber Aufgabe der Erfindung, ein Verfahren zur Herstellung von aromatischen oder aliphatischen Isocyanaten durch thermische Spaltung der entsprechenden Carbamate zur Verfügung zu stellen, das hohe Ausbeuten und einen niedrigen Anteil an Nebenprodukten bei wenig Fouling ermöglicht.

Die Lösung besteht in einem Verfahren zur Herstellung von Isocyanaten durch thermische Spaltung von Carbamaten und destillative Auftrennung des Reaktionsgemisches der Carbamatspaltung, enthaltend das entsprechende Isocyanat und den entsprechenden Alkohol in einer Kolonne mit einem Verstärkungsteil und einem Abtriebsteil, wobei das Carbamat zwischen dem Verstärkungsteil und dem Abtriebsteil aufgegeben wird, und das Isocyanat als Bestandteil des Sumpfstromes und der Alkohol als Bestandteil des Kopfstromes der Kolonne abgezogen wird, in Gegenwart eines inerten Lösungsmittels, das dadurch gekennzeichnet ist, dass als inertes Lösungsmittel ein Zwischensieder mit einem Siedepunkt zwischen dem Siedepunkt des Isocyanats und dem Siedepunkt des Alkohols unter den Betriebsbedingungen der Carbamatspaltung eingesetzt wird, der im oberen Bereich des Verstärkungsteils als externer Rücklauf flüssig, in einer Reinheit > 95 Gew.-%, bezogen auf das Gesamtgewicht des externen Rücklaufs, und im Abtriebsteil an einer oder mehreren Stellen als gasförmiger, überhitzter Strom eingespeist wird.

Der Zusatz von inerten Lösungsmitteln zur Verdünnung des Carbamat-Spaltgases und damit Reduzierung der Bildung von Nebenkomponenten ist bekannt. Inert bedeutet, wie üblich, dass das Lösungsmittel unter Verfahrensbedingungen nicht mit den Komponenten des Reaktionsgemisches reagiert.

Es wurde gefunden, dass durch die Zugabe eines inerten Lösungsmittels, das ein Zwischensieder ist, d.h. das einen Siedepunkt zwischen dem Siedepunkt des Isocyanats und dem Siedepunkt des Alkohols unter Betriebsbedingungen aufweist, und der darüber hinaus in hoher Reinheit, d.h. in einer Reinheit > 95 Gew-%, eingesetzt wird, gleichzeitig erreicht werden kann, dass am Kopf der Kolonne der dem Carbamat entsprechende Alkohol in hoher Reinheit gewonnen werden kann, ohne dass der Rücklauf Alkohol enthält. Indem somit die Anwesenheit von Alkohol im flüssigen Rücklauf in der Kolonne vermindert oder ausgeschlossen wird, wird die Rückreaktion der Spaltprodukte zum Carbamat im Verstärkungsteil verlangsamt. Würde hingegen der Rücklauf durch reinen Alkohol erzeugt werden, so würde der Verstärkungsteil erhöhte Konzentrationen an Alkohol aufweisen. Um eine maximale Gleichgewichtsverschiebung in Richtung der Spaltprodukte Isocyanat und Alkohol zu erreichen, muss jedoch der Alkohol so schnell wie möglich aus dem System entfernt werden. Weiterhin ist es demnach vorteilhaft, dass im Verstärkungsteil druckverlustarme Hochleistungspackungen mit einem Holdup von weniger als 5% auf das Leerrohrvolumen eingesetzt werden, um die Reaktion zu verlangsamen.

Im Abtriebsteil der Kolonne erfolgt überwiegend die Carbamatspaltung.

Die Reaktion ist stark endotherm. Sie muss in kurzer Verweilzeit, mit möglichst wenig Rückvermischung, erfolgen.

Wird die Spalttemperatur sehr hoch gewählt, beispielsweise über 300 °C, so kann der Abtriebsteil einem oder mehreren hintereinander geschalteten Fallfilmverdampfer umfassen. Ist die Spalttemperatur jedoch niedriger, beispielsweise unter 300 °C, so werden bevorzugt Verweilzeitböden eingesetzt, um den Zielumsatz zu erreichen.

Der Abtriebsteil der Kolonne, worin die Carbamatspaltung durchgeführt wird, ist demnach bevorzugt als Fallfilmverdampfer oder als Verweilzeitböden ausgebildet.

Als Verweilzeitboden können z. B. Tunnel-, Thormann- oder Lord-Böden, bevorzugt Lord-Böden beispielsweise wie in EP1493475B1 beschrieben, gewählt werden. Die Böden können auch von unten mit Dampf beheizt werden, um zusätzlich Energie für die endotherme Reaktion zur Verfügung zu stellen.

Der Abtriebsteil wird bevorzugt so dimensioniert, dass am Sumpfverdampfer das gewünschte Maß der Carbamatspaltung erreicht wird, in der Regel mehr als 99 % des eingesetzten Carbamats.

Um die Verweilzeit zu vergrößern, ist weiterhin eine sequentielle Abfolge von Fallfilmverdampfern mit Neuverteilung über einen Verteiler auf den jeweils nächsten Fallfilmverdampfer möglich.

Vorteilhaft kann der eine oder die mehreren hintereinander geschalteten Fallfilmverdampfer oder die Verweilzeitböden katalytisch beschichtet sein.

Werden Verweilzeit- oder insbesondere Lord-Böden eingesetzt, so kann ein immobilisierter heterogener Katalysator oder ein Suspensionskatalysator auf den Böden verwendet werden.

Die Carbamatspaltung wird insbesondere bei einer Temperatur zwischen 210 °C und 400 °C durchgeführt.

In dem erfindungsgemäßen Verfahren können übliche Carbamate (auch als Carbamidsäureester oder Urethane bezeichnet), bevorzugt Biscarbamate, zur Spaltung eingesetzt werden. Diese Carbamate basieren üblicherweise auf der allgemein bekannten Umsetzung von Aminen, vorzugsweise von Di- oder Polyaminen, bevorzugt von Diaminen, mit Harnstoff und mindestens einem Alkohol.

Besonders bevorzugt erfolgt die Umsetzung des Diamins oder Polyamins zum entsprechenden Carbamat mit Carbonaten in Gegenwart von Alkoholaten als Base wie es in WO 2009/115538 beschrieben wird.

Als Alkohole zur Herstellung der Carbamate eignen sich prinzipiell alle aliphatischen Alkohole. Vorzugsweise werden solche ausgewählt, deren Siedepunkte sich hinreichend vom Siedepunkt der Isocyanate unterscheiden, um eine optimale Trennung zu gewährleisten. Besonders bevorzugt werden zur Herstellung des Carbamats aliphatische Monohydroxyalkohole mit 1 bis 4 C-Atomen pro Molekül, d.h. Methanol, Ethanol, Propanol, Isopropanol, n-Butanol und/oder Isobutanol, eingesetzt. Bevorzugt sind weiterhin Alkohole mit mindestens einem Sauerstoffheteroatom, insbesonder 2-Methoxyethanol, 2-Ethoxyethanol, 2-Propoxyethanol, 2-Butoxyethanol, 2-Methoxy-1-propanol und/oder 1-Methoxy-2-propanol.

Als Amine werden bevorzugt 2,4- und/oder 2,6-Toluylendiamin (TDA), 2,2', 2,4'-und/oder 4,4'-Diaminodiphenylmethan (MDA) und/oder höhere Homologe (Polyphenylenpolymethylenpolyamine, pMDA), 1,6-Hexamethylendiamin (HDA), 1-Amino-3,3,5-trimethyl-5-aminomethylcyclohexan (im Folgenden auch als Isophorondiamin, IPDA bezeichnet), 1,5- und/oder 1,8-Diaminonaphthalin, 4,4'-Diaminodiphenyl, 1,3- und/oder 1,4-Diaminobenzol, 2,4- und/oder 2,6-Hexahydrotoluylendiamin und/oder 4,4'-, 2,4'- und/oder 2,2'-Dicyclohexylmethandiamin eingesetzt. Die Strukturen der eingesetzten Amine determinieren die Strukturen der nach der thermischen Spaltung erhältlichen Isocyanate. Besonders bevorzugt basieren die eingesetzten Urethane auf 2,4- und/oder 2,6-Toluylendiamin (TDA), 2,2'-, 2,4'- und/oder 4,4'-Diaminodiphenylmethan (MDA) und/oder höhere Homologe (Polyphenylenpolymethylenpolyamine, pMDA), 1,6-Hexamethylendiamin (HDA), Isophorondiamin (IPDA) und/oder 1,5-Diaminonaphthalin als Amin-Komponente und Methanol, n-Propanol, Isopropanol, n-Butanol, oder insbesondere Isobutanol oder 2-Methoxyethanol als Alkohol.

Insbesondere werden demnach die folgenden Diurethane oder Polyurethane zur Spaltung eingesetzt: 2,4- und/oder 2,6-Toluylendiisobutylurethan, 2,4- und/oder 2,6-Toluylendimethoxyethylurethan, 2,4- und/oder 2,6-Toluylendipropylurethan, 2,4- und/oder 2,6-Toluylendimethylurethan, 1,5-Naphthylendiisobutylurethan, 1,5-Naphthylendimethoxyethylurethan, 1,5-Naphthylendipropylurethan, 1,5-Naphthylendimethylurethan, 4,4'-, 2,4'- und/oder 2,2'-Diphenylmethandiisobutylurethan, 4,4'-, 2,4'- und/oder 2,2' Diphenylmethandimethoxyethylurethan, 4,4'-, 2,4'- und/oder 2,2'-Diphenylmethandipropylurethan, 4,4'-, 2,4'- und/oder 2,2'-Diphenylmethandimethylurethan, Polyphenylenpolymethylenpoylmethoxyethylurethan, Polyphenylenpolymethylenpoylmethylurethan, Polyphenylenpolymethylenpolypropylurethan, Polyphenylenpolymethylenpolyisobutylurethan, 1,6-Hexamethyldiisobutylurethan, 1,6-Hexamethylendimethoxyethylurethan, 1,6-Hexamethylendipropylurethan, 1,6-Hexamethylendimethylurethan, Isophorondiisobutylurethan, Isophorondimethoxyethylurethan, Isophorondipropylurethan und/oder Isophorondimethylurethan, wobei auch Mischungen der genannten Urethane zur Spaltung eingesetzt werden können.

Besonders bevorzugt werden die folgenden Isocyanate durch thermische Spaltung der entsprechenden Diurethane hergestellt: 2,4- und/oder 2,6-Toluylendiisocyanat (TDI), 2,2'-, 2,4'- und/oder 4,4'-Diisocyanatodiphenylmethan (MDI), Polyphenylenpolymethylenpolyisocyanat (pMDI), 1,6-Hexamethylendiisocyanat (HDI), 1-Amino-3,3,5-trimethyl-5-aminomethylcyclohexan (Isophorondiisocanat, IPDI) und/oder 1,5-Diisocyanatonaphthalin (NDI).

Bevorzugt wird als inertes Lösungsmittel ein solches eingesetzt, das einen Siedepunkt zwischen 70 und 350 °C unter Normalbedingungen aufweist.

Besonders bevorzugt ist das inerte Lösungsmittel ein Zwischensieder mit einem Siedepunkt zwischen 100 °C und 250 °C.

Geeignete inerte Lösungsmittel sind insbesondere Tetralin, Diphyl (Gemisch aus Biphenyl und Diphenylether), Biphenyl, Diphenylether, die isomeren Benzyltoluole, die isomeren Dibenzyltoluole, Dibenzylether, die isomeren Trichlorbenzole, die isomeren Dichlortoluole, die isomeren Diethyltoluole, die isomeren Diethylbenzole, die isomeren Dipropylbenzole, die isomeren Diisopropylbenzole und/oder die isomeren Tetramethylbenzole.

In einer bevorzugten Ausführungsform wird zusätzlich inertes Lösungsmittel an der Feedstelle für das Carbamat, das der Spaltung zugeführt wird, mit eingespeist. Das Lösungsmittel kann flüssig, tauend (gerade total verdampft), oder bevorzugt gasförmig zugeführt werden.

Besonders bevorzugt handelt es sich hierbei um dasselbe Lösungsmittel, das auch als externer Rücklauf flüssig auf den Verstärkungsteil und als gasförmiger, überhitzter Strom im unteren Bereich des Abtriebsteils eingespeist wird. Zusätzlich kann tauender oder überhitzter Wasserdampf an mehreren Stellen im Abtriebsteil der Kolonne eingebracht werden.

Vorteilhaft wird der aus dem Verstärkungsteil abgezogene Brüden gequencht.

Erfindungsgemäß ist es weiterhin wesentlich, dass der gequenchte Brüden nicht aus reinem Alkohol besteht, sondern 10 bis 95 Vol.% Lösungsmittel, bevorzugt 50 bis 95 Vol.-% Lösungsmittel, enthält.

Die Erfindung wird im Folgenden anhand einer Zeichnung sowie von Ausführungsbeispielen näher erläutert.

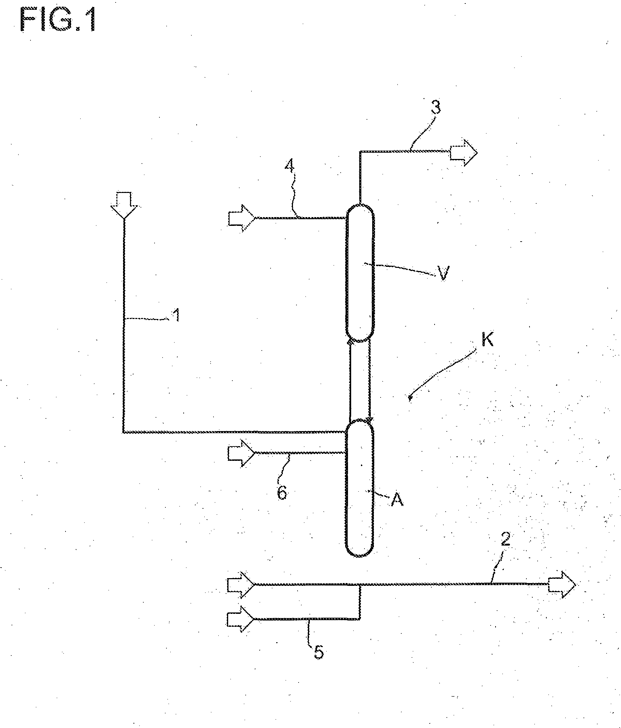

Die Figur 1 zeigt die schematische Darstellung einer Anlage zur Durchführung des erfindungsgemäßen Verfahrens.

Einer Kolonne K mit Verstärkungsteil V und Abtriebsteil A wird im mittleren Bereich derselben ein Strom 1 des zu spaltenden Carbamats zugeführt.

Aus dem Sumpf des Abtriebsteils A wird ein Strom 2, enthaltend das entsprechende Isocyanat, und über Kopf des Verstärkungsteils V ein Strom 3, enthaltend den entsprechenden Alkohol, abgezogen.

Im oberen Bereich des Verstärkungsteils V wird ein Strom 4, enthaltend einen Zwischensieder mit einem Siedepunkt zwischen dem Siedepunkt des über Sumpf abgezogenen Isocyanats und dem Siedepunkt des über Kopf abgezogenen Alkohols, mit einer Reinheit > 95 %, flüssig zugeführt.

Im unteren Bereich des Abtriebsteils A wird ein Strom 5, enthaltend den gasförmigen, überhitzten Zwischensieder, zugeführt.

In der in der Figur dargestellten bevorzugten Ausführungsform wird zusätzlich ein Strom desselben Zwischensieders, Strom 6, zusammen mit dem zu spaltenden Carbamat, Strom 1, dem mittleren Bereich der Kolonne K zugeführt.

### Ausführungsbeispiele

### Beispiel 1a: Spaltung oberhalb von 300°C (erfindungsgemäß)

Es wird eine Spaltapparatur wie in Figur 1 beschrieben, eingesetzt. Die Apparatur verfügt über 6 theoretische Trennstufen im Verstärkungsteil V und einer theoretischen Trennstufe im Abtriebsteil A. Der Abtriebsteil A besteht aus einem stehenden Rohrbündel.

2,5 kg/h einer 20 gew.-%igen Lösung von 2,4-Toluylen-bis(O-diisobutylcarbamat) (Strom 1) in 1,2,4-Trichlorbenzol werden mit 120 °C in die Spaltapparatur oberhalb des Abtriebsteils gefahren.

Die Kolonne wird bei einem Druck von 10 bar betrieben. Am Kolonnenfeed werden zusätzlich 1,0 kg/h bei 339 °C/10 bar siedendes 1,2,4-Trichlorbenzol (Strom 6) zudosiert. Am Kolonnenkopf werden 2,3 kg/h 1,2,4-Trichlorbenzol (Strom 4) mit einer Reinheit von 99,5 % bei 300 °C auf den Flüssigverteiler gegeben. Die Sumpftemperatur beträgt 348 °C. Am Kolonnensumpf werden 0,053 kg/h N₂ (Strom 5) und 0,4 kg/h 1,2,4-Trichlorbenzoldampf (350°C) zum Strippen des Alkohols zugeführt. Am Sumpfausgang der Kolonne werden 3,6 kg/h einer Mischung (Strom 2) abgezogen, enthaltend 248 g/h 2,4-Toluylendiisocyanat (TDI), 149 ppm Semicarbamate und 23,3 g/h hochsiedende Nebenkomponenten. Der 1,2,4-Trichlorbenzolgehalt beträgt 92,4%.

Am Kopf der Apparatur werden bei 318 °C 2,66 kg/h einer Mischung enthaltend 8,3 Gew.% Isobutanol und 0,1 Gew.-% 2,4-TDI sowie weniger als 100 ppm an Semicarbamaten abgezogen.

Das an zwei Stellen eingespeiste 1,2,4-Trichlorbenzol (Ströme 6, 4) hat jeweils dieselbe Zusammensetzung (Reinheit: 99,5 %).

Die Gesamtausbeute an 2,4-TDI in Sumpf-und Kopfprodukt beträgt 92,9 %.

### Beispiel 1b: Spaltung oberhalb von 300°C (zum Vergleich)

Es wird eine Spaltapparatur wie in Figur 1 beschrieben, eingesetzt. Die Apparatur verfügt über 6 theoretische Trennstufen im Verstärkungsteil V und einer theoretischen Trennstufe im Abtriebsteil A. Der Abtriebsteil A besteht aus einem stehenden Rohrbündel.

2,5 kg/h einer 20 gew.-%igen Lösung von 2,4-Toluylen-bis(O-diisobutylcarbamat) (Strom 1) in 1,2,4-Trichlorbenzol werden mit 120 °C in die Spaltapparatur oberhalb des Abtriebsteils gefahren.

Die Kolonne wird bei einem Druck von 10 bar betrieben. Am Kolonnenfeed werden zusätzlich 1,0 kg/h bei 339 °C/10 bar siedendes 1,2,4-Trichlorbenzol (Strom 6) zudosiert. Am Kolonnenkopf wird jedoch kein 1,2,4-Trichlorbenzol zugegeben, sondern ein Kondensator mit dem Rücklaufverhältnis von 0,8 betrieben. Die Sumpftemperatur beträgt 348 °C. Am Kolonnensumpf werden 0,12 kg/h N₂ (Strom 5) und 0,4 kg/h 1,2,4-Trichlorbenzol (350 °C) zum Strippen des Alkohols zugeführt. Am Sumpfausgang der Kolonne werden 2,3 kg/h einer Mischung (Strom 2) abgezogen, enthaltend 214 g/h 2,4-TDI, 15 ppm Semicarbamate und 34,5 g/h hochsiedende Nebenkomponenten. Der 1,2,4-Trichlorbenzolgehalt beträgt 89,2%.

Am Kopf der Apparatur werden bei 296 °C 1,71 kg/h einer Mischung enthaltend 12,9 Gew.% Isobutanol und 1,7 Gew.-% 2,4-TDI sowie weniger als 100 ppm an Semicarbamaten abgezogen. Die Gesamtausbeute an 2,4-TDI in Sumpf und Kopf beträgt lediglich 89,5%.

### Beispiel 2: Spaltung unterhalb von 300°C (erfindungsgemäß)

Es wird eine Spaltapparatur wie in Figur 1 beschrieben eingesetzt. Die Apparatur verfügt über 10 theoretische Trennstufen im Verstärkungsteil V und 30 theoretische Trennstufen im Abtriebsteil A. Der Abtriebsteil verfügt über keinen eigenen Verdampfer. Die Apparatur verfügt im Verstärkungsteil über druckverlustarme Gewebepackungen mit einem Holdup von weniger als 5% bezogen auf den Leerrohrquerschnitt. Der Abtriebsteil besteht aus 30 Verweilzeitböden wie in EP 1493475 B1 beschrieben.

1,0 kg/h einer 50 gew.-%igen Lösung von 2,4-Toluylen-bis(O-diisobutylcarbamat) (Strom 1) in 1,2,4-Trichlorbenzol werden mit 180 °C der Spaltapparatur oberhalb des Abtriebsteils (Stufe 30 von unten) zugeführt.

Die Kolonne wird bei einem Druck von 4 bar betrieben. Am Kolonnenfeed (Stufe 30 von unten) werden zusätzlich 2,37 kg/h bei 281 °C überhitztes gasförmiges 1,2,4-Trichlorbenzol (Strom 6) zudosiert. Weiterhin werden auf Stufe 15 von unten 0,49 kg/h überhitztes 1,2,4-Trichlorbenzol bei 281 °C zudosiert. Am Kolonnenkopf werden 0,95 kg/h 1,2,4-Trichlorbenzol (Strom 4) mit einer Reinheit von 99,5 % bei 250 °C auf den Flüssigverteiler gegeben. Die Sumpftemperatur beträgt 270 °C. Am Kolonnensumpf werden 0,03 kg/h N₂ (Strom 5) zum Strippen des Alkohols und 1,17 kg/h gasförmiges 1,2,4-Trichlorbenzol bei 281 °C zur Energiezufuhr eingespeist. Am Sumpfausgang der Kolonne werden 3,8 kg/h einer Mischung (Strom 2) abgezogen, enthaltend 5,1 Gew.-% 2,4-TDI.

Am Kopf der Apparatur werden bei 264 °C 2,0 kg/h einer Mischung enthaltend 9,2 Gew.-% Isobutanol und 0,05 Gew.-% 2,4-TDI sowie weniger als 100 ppm an Semicarbamaten abgezogen.

Das an vier Stellen eingespeiste 1,2,4-Trichlorbenzol (Rücklauf, Strom 6, Stufe 15, Sumpf) hat jeweils dieselbe Zusammensetzung (Reinheit: 99,5 %).

In dieser bevorzugten Ausführung werden weder Verdampfer noch Kondensatoren eingesetzt. Ein Wärmeübertrager-Fouling im Spalter ist damit ausgeschlossen.

## Patentansprüche

1. Verfahren zur Herstellung von Isocyanaten durch thermische Spaltung von Carbamaten und destillative Auftrennung des Reaktionsgemisches der Carbamatspaltung, enthaltend das entsprechende Isocyanat und den entsprechenden Alkohol, in einer Kolonne (K) mit einem Verstärkungsteil (V) und einem Abtriebsteil (A), wobei das Carbamat (1) zwischen dem Verstärkungsteil (V) und dem Abtriebsteil (A) aufgegeben wird, und das Isocyanat als Bestandteil des Sumpfstromes (2) und der Alkohol als Bestandteil des Kopfstromes (3) der Kolonne (K) abgezogen wird, in Gegenwart eines inerten Lösungsmittels, **dadurch gekennzeichnet, dass** als inertes Lösungsmittel ein Zwischensieder mit einem Siedepunkt zwischen dem Siedepunkt des Isocyanats und dem Siedepunkt des Alkohols unter den Betriebsbedingungen der Carbamatspaltung eingesetzt wird, der im oberen Bereich des Verstärkungsteils (V) als externer Rücklauf (4) flüssig, in einer Reinheit > 95 Gew.-%, bezogen auf das Gesamtgewicht des externen Rücklaufs (4) und im Abtriebsteil (A) an einer oder mehreren Stellen als gasförmiger, überhitzter Strom (5) eingespeist wird.

2. Verfahren nach Anspruch 1, **dadurch gekennzeichnet, dass** ein inertes Lösungsmittel in einem Siedepunkt im Bereich zwischen 70 und 350 °C unter Normaldruck eingesetzt wird.

3. Verfahren nach Anspruch 2, **dadurch gekennzeichnet, dass** ein inertes Lösungsmittel mit einem Siedepunkt zwischen 100 und 250°C eingesetzt wird.

4. Verfahren nach Anspruch 3, **dadurch gekennzeichnet, dass** der Alkohol ein aliphatischer Monohydroxyalkohol, insbesondere Methanol, Butanol, Isobutanol, Methoxyethanol, Butoxyethanol oder 2-Methoxy-1-propanol ist.

5. Verfahren nach einem der Ansprüche 1 bis 4, **dadurch gekennzeichnet, dass** der Abtriebsteil (A) der Kolonne (K) als Fallfilmverdampfer ausgebildet ist.

6. Verfahren nach Anspruch 5, **dadurch gekennzeichnet, dass** der Abtriebsteil (A) der Kolonne (K) mehrere hintereinander geschaltete Fallfilmverdampfer mit dazwischen angeordneten Verteilern aufweist.

7. Verfahren nach Anspruch 5 oder 6, **dadurch gekennzeichnet, dass** der eine oder die mehreren hintereinander geschalteten Fallfilmverdampfer katalytisch beschichtet ist/sind.

8. Verfahren nach einem der Ansprüche 1 bis 7, **dadurch gekennzeichnet, dass** ein Strom (6) eines inerten Lösungsmittels an der Feedstelle für das Carbamat (1) der Kolonne (K) zugeführt wird.

9. Verfahren nach Anspruch 8, **dadurch gekennzeichnet, dass** das inerte Lösungsmittel, das als Strom (6) an der Feedstelle für das Carbamat (1) der Kolonne (K) zugeführt wird, dieselbe Substanz enthält, die als Zwischensieder als externer Rücklauf (4) auf den oberen Bereich des Verstärkungsteils (V) sowie als gasförmiger, überhitzter Strom (5) im Abtriebsteil (A) zugeführt wird.

10. Verfahren nach einem der Ansprüche 1 bis 9, **dadurch gekennzeichnet, dass** der den dem Carbamat entsprechende Alkohol enthaltende Kopfstrom (3) gasförmig abgezogen und unmittelbar nach dem Abziehen desselben gequencht wird.

11. Verfahren nach einem der Ansprüche 1 bis 10, **dadurch gekennzeichnet, dass** 50 bis 100 % des bei der Spaltung des Carbamats entstehenden Isocyanats über Sumpf abgezogen werden.

12. Verfahren nach einem der Ansprüche 1 bis 11, **dadurch gekennzeichnet, dass** die Spaltung des Carbamats bei einer Betriebstemperatur zwischen 210 °C und 400 °C durchgeführt wird.

13. Verfahren nach einem der Ansprüche 1 bis 4 oder 8 bis 12, **dadurch gekennzeichnet, dass** im Abtriebsteil (A) der Kolonne (K) Lord-Böden als Verweilzeitböden eingesetzt werden.

14. Verfahren nach Anspruch 13, **dadurch gekennzeichnet, dass** die Verweilzeitböden mit Katalysator beschickt sind.

## Claims

1. A process for preparing isocyanates by thermal dissociation of carbamates and separation by distillation of the reaction mixture from the carbamate dissociation, comprising the corresponding isocyanate and the corresponding alcohol, by distillation in a column (K) having an enrichment section (V) and a stripping section (A), where the carbamate (1) is introduced between the enrichment section (V) and the stripping section (A) and the isocyanate is taken off as a constituent of the bottom stream (2) and the alcohol is taken off as a constituent of the overhead stream (3) from the column (K), in the presence of an inert solvent, wherein an intermediate boiler having a boiling point between the boiling point of the isocyanate and the boiling point of the alcohol under the operating conditions of the carbamate dissociation is used as inert solvent and is fed as external runback (4) in liquid form in a purity of > 95% by weight, based on the total weight of the external runback (4), in the upper region of the enrichment section (V) and as gaseous, superheated stream (5) into the stripping section (A) at one or more points.

2. The process according to claim 1, wherein an inert solvent having a boiling point in the range from 70 to 350°C under atmospheric pressure is used.

3. The process according to claim 2, wherein an inert solvent having a boiling point in the range from 100 to 250°C is used.

4. The process according to claim 3, wherein the alcohol is an aliphatic monohydroxy alcohol, in particular methanol, butanol, isobutanol, methoxyethanol, butoxyethanol or 2-methoxy-1-propanol.

5. The process according to any of claims 1 to 4, wherein the stripping section (A) of the column (K) is configured as a falling film evaporator.

6. The process according to claim 5, wherein the stripping section (A) of the column (K) has a plurality of falling film evaporators connected in series with distributors arranged in between.

7. The process according to claim 5 or 6, wherein the one or more falling film evaporators connected in series is/are catalytically coated.

8. The process according to any of claims 1 to 7, wherein a stream (6) of an inert solvent is fed into the column (K) at the feed point for the carbamate (1).

9. The process according to claim 8, wherein the inert solvent which is fed as stream (6) into the column (K) at the feed point for the carbamate (1) comprises the same substance which is fed as intermediate boiler as external runback (4) to the upper region of the enrichment section (V) and as gaseous, superheated stream (5) into the stripping section (A).

10. The process according to any of claims 1 to 9, wherein the overhead stream (3) comprising the alcohol corresponding to the carbamate is taken off in gaseous form and is quenched immediately after being taken off.

11. The process according to any of claims 1 to 10, wherein from 50 to 100% of the isocyanate formed in the dissociation of the carbamates is taken off via the bottom.

12. The process according to any of claims 1 to 11, wherein the dissociation of the carbamate is carried out at an operating temperature in the range from 210°C to 400°C.

13. The process according to any of claims 1 to 4 or 8 to 12, wherein Lord trays are used as residence trays in the stripping section (A) of the column (K).

14. The process according to claim 13, wherein the residence trays are provided with catalyst.

## Revendications

1. Procédé pour la production d'isocyanates par coupure thermique de carbamates et fractionnement par distillation du mélange réactionnel de la coupure de carbamate, contenant l'isocyanate correspondant et l'alcool correspondant, dans une colonne (K) comportant un tronçon de concentration (V) et un tronçon d'épuisement (A), le carbamate (1) étant introduit entre le tronçon de concentration (V) et le tronçon d'épuisement (A), et l'isocyanate étant déchargé en tant que composant du courant de pied (2) et l'alcool étant évacué en tant que composant du courant de tête (3) de la colonne (K), en présence d'un solvant inerte, **caractérisé en ce qu'**on utilise comme solvant inerte un composé à point d'ébullition intermédiaire, ayant un point d'ébullition compris entre le point d'ébullition de l'isocyanate et le point d'ébullition de l'alcool dans les conditions de fonctionnement de la coupure de carbamate, qui est introduit à l'état liquide dans la zone supérieure du tronçon de concentration (V) en tant que courant de recyclage externe (4), à un degré de pureté > 95 % en poids, par rapport au poids total du courant de recyclage externe (4) et dans le tronçon d'épuisement (A) en un ou plusieurs points en tant que courant gazeux surchauffé (5).

2. Procédé selon la revendication 1, **caractérisé en ce qu'**on utilise un solvant inerte ayant un point d'ébullition dans la plage comprise entre 70 et 350 °C sous la pression normale.

3. Procédé selon la revendication 2, **caractérisé en ce qu'**on utilise un solvant inerte ayant un point d'ébullition compris entre 100 et 250 °C.

4. Procédé selon la revendication 3, **caractérisé en ce que** l'alcool est un alcool monohydrique, en particulier le méthanol, le butanol, l'isobutanol, le méthoxyéthanol, le butoxyéthanol ou le 2-méthoxy-1-propanol.

5. Procédé selon l'une quelconque des revendications 1 à 4, **caractérisé en ce que** le tronçon d'épuisement (A) de la colonne (K) est conçu sous forme d'évaporateur à film descendant.

6. Procédé selon la revendication 5, **caractérisé en ce que** le tronçon d'épuisement (A) de la colonne (K) comporte plusieurs évaporateurs à film descendant raccordés les uns à la suite des autres, avec des distributeurs disposés entre eux.

7. Procédé selon la revendication 5 ou 6, **caractérisé en ce que** ledit un ou lesdits plusieurs évaporateurs à film descendant raccordés les uns à la suite des autres est/sont revêtu(s) avec un revêtement catalytique.

8. Procédé selon l'une quelconque des revendications 1 à 7, **caractérisé en ce qu'**on envoie un courant (6) d'un solvant inerte au niveau du point d'alimentation pour le carbamate (1) de la colonne (K).

9. Procédé selon la revendication 8, **caractérisé en ce que** le solvant inerte qui est envoyé en tant que courant (6) au niveau du point d'alimentation pour le carbamate (1) de la colonne (K) contient la même substance qui est envoyée en tant que composé à point d'ébullition intermédiaire sous forme de courant de recyclage externe (4) à la zone supérieure du tronçon de concentration (V) ainsi qu'en tant que courant gazeux surchauffé (5) dans le segment d'épuisement (A).

10. Procédé selon l'une quelconque des revendications 1 à 9, **caractérisé en ce que** le courant de tête (3) contenant l'alcool correspondant au carbamate est déchargé à l'état gazeux et refroidi brusquement immédiatement après l'évacuation de celui-ci.

11. Procédé selon l'une quelconque des revendications 1 à 10, **caractérisé en ce que** 50 à 100 % de l'isocyanate formé lors de la coupure du carbamate sont déchargés par le pied.

12. Procédé selon l'une quelconque des revendications 1 à 11, **caractérisé en ce que** la coupure du carbamate est effectuée à une température de fonctionnement comprise entre 210 °C et 400 °C.

13. Procédé selon l'une quelconque des revendications 1 à 4 ou 8 à 12, **caractérisé en ce que** dans le tronçon d'épuisement (A) de la colonne (K) on utilise des plateaux Lord en tant que plateaux à temps de séjour.

14. Procédé selon la revendication 13, **caractérisé en ce que** les plateaux à temps de séjour sont revêtus de catalyseur.
